# EUROPEAN PATENT SPECIFICATION

(11) **EP 3 258 887 B1**
(45) Date of publication and mention of the grant of the patent: **02.04.2025**
(21) Application number: 16721921.1
(22) Date of filing: 16.02.2016
(51) Int. Cl.: A61F 2/30

(54) **SPACER DEVICE FOR TREATING AN INFECTED SEAT INSIDE THE HUMAN BODY**
ABSTANDSVORRICHTUNG ZUR BEHANDLUNG EINES INFIZIERTEN SITZES IM INNEREN DES MENSCHLICHEN KÖRPERS
DISPOSITIF D'ÉCARTEMENT POUR TRAITER UN SIÈGE INFECTÉ À L'INTÉRIEUR DU CORPS HUMAIN

(30) Priority: 16.02.2015 IT BO20150066
(43) Date of publication of application: 27.12.2017
(73) Proprietor: TECRES S.P.A., 37066 Sommacampagna (VR) (IT)
(72) Inventor: MAGAGNOLI, Augusto, 48015 Cervia (Ravenna) (IT)
(74) Representative: Feltrinelli, Secondo Andrea
(86) International application number: PCT/IB2016/000134
(87) International publication number: WO 2016/132200

(56) References cited:
- EP-A1- 0 025 814
- WO-A1-2012/120480
- WO-A1-2013/041905
- US-A- 5 549 699
- US-A1- 2007 016 163
- US-A1- 2009 157 189
- US-B1- 6 245 111

## Description

### TECHNICAL FIELD OF THE INVENTION

The present invention relates to a spacer device, of temporary and disposable type, for the treatment of a bone or joint seat of the human body affected by infection.

The present invention also relates to a method for making one such spacer device.

### STATE OF THE PRIOR ART

It is known that the prostheses implanted inside the human body can be subjected to infections.

In such event, the infected prosthesis must be removed from the implant site and, before the implant of a new prosthesis, the infection must be eradicated.

During such step, spacer devices are normally used for the purpose of substantially maintaining unchanged the shape of the bone seat or of the joint seat in which the new prosthesis will be implanted.

Such procedure is known as "two-step treatment" for the removal of an infected prosthesis and the implant of a new prosthesis.

The spacer devices normally used can have an outer surface of porous type, which can possibly be impregnated with one or more pharmaceutical or medical substances to be released in the human body, at the anatomic area where their implant is provided.

In such spacer devices, the quantity of pharmaceutical or medical substance that can possibly be impregnated along the porous outer surface is limited by the depth and by the extension of the surface itself. In such case, the spacer device might not be able to ensure a release of the pharmaceutical or medical substance for a period equal to that necessary for the complete healing of the infected site.

In addition, in one such spacer device, it is difficult to apply, along specific portions thereof, two or more pharmaceutical or medical substances that are different from each other, even while maintaining such substances separate.

There are then preformed spacer devices which are produced by casting already-antibiotic bone cement in a mold up to the hardening thereof, and extracting the hardened spacer device from the mold; such device is subsequently worked or finished in accordance with the requirements.

Alternatively, the surgeon can himself make a spacer during the operating phase by using molds - usually made of silicone of suitable geometry - which are filled with antibiotic bone cement, to which a further antibiotic different from the first is possibly added. Once the polymerization has taken place, the surgeon extracts the spacer from the silicone mold, aided by the flexible nature of the silicone material, and then proceeds with the implant, possibly finishing the spacer, if necessary.

Also in this case, it is difficult for a surgeon to arrange pharmaceutical or medical substances in specific portions of the spacer device, since the antibiotic bone cement cast in the mold is freely arranged inside the same, filling the cavities thereof.

There is therefore the need, for the surgeon, to have a spacer device in which it is possible to add one or more pharmaceutical or medical substances along specific portions of the device itself, in the scope of a solution that is easy to actuate.

In addition, there is also the need to provide a spacer device that ensures the release of one or more pharmaceutical or medical substances for the entire period provided for the treatment of the infected site.

Simultaneously, these possibilities are associated with the need to provide, in any case, a temporary and/or disposable spacer device with predefined and correct shape and size, without the risk that the surgeon - having to make the spacer device directly *in situ* - will obtain a shape that is irregular or incompatible with the actual anatomic needs of the patient, or in any case to be finished and worked before implant.

Document US2007/0016163 discloses implantable devices for use in the treatment of osteonecrosis, which includes a plurality of reservoirs with a release system. US6245111 discloses a prosthetic device and an endoskeleton for the realization of a prosthetics device. US2009/0157189 discloses a modular spacer mold for forming a temporary implant comprising a head component mold and a stem component mold. WO2013/041905A1 discloses a modular spacer device for the temporary replacement of articular prostheses with ribs allowing to hold bone cement. These ribs can be webbed.

### OBJECTS OF THE INVENTION

The task of the present invention is to improve the state of the prior art.

In the scope of such technical task, one object of the present invention is to provide a spacer device for the treatment of an infected site of the human body that is easy to use, for the release of at least one pharmaceutical or medical substance in a bone or joint seat to be treated.

A further object of the present invention is to provide a spacer device for the release of at least one pharmaceutical or medical substance in specific portions of the bone or joint seat of the human body with which it is associated, possibly even for long time periods.

Another object of the present invention is to provide a spacer device comprising at least one coupling surface configured for facilitating a stable connection between the spacer device itself and the bone or joint seat with which it is associated.

In accordance with one aspect of the present invention, a spacer device is provided, according to claim 1.

The dependent claims refer to preferred and advantageous embodiments of the invention.

### BRIEF DESCRIPTION OF THE DRAWINGS

Further characteristics and advantages of the present invention will be clearer from the detailed description of a preferred but not exclusive embodiment of a spacer device, illustrated as a non-limiting example in the enclosed drawing tables, in which:
figure 1 is a perspective view of the base or lower part of a spacer device according to the present invention;
figure 2 is a perspective view of an articular or upper part of a spacer device according to figure 1;
figure 3 is a perspective view of the upper part of a further embodiment of a spacer device according to the present invention;
figure 4 is a perspective view of the device according to figure 3 from another angle;
figure 5 is a perspective view of the lateral portion of a further embodiment of a spacer device according to the present invention;
figure 6 is a detailed view of a lateral section of a spacer device according to the present invention, at a coupling surface thereof.

### EMBODIMENTS OF THE INVENTION

As is known, a spacer device is provided for being implanted in a bone or joint seat of the human body, typically in substitution of an infected prosthesis.

The spacer device according to the present invention is of temporary and/or disposable type.

With the term "temporary" it is intended that, once its curative function has been completed, together with its maintenance of the space of the bone seat or joint seat, the spacer device will be removed from the zone in question and substituted for example with a permanent prosthesis.

For such purpose, the spacer device performs the function of maintenance of the joint spaces as well as of treatment of the bone infection by releasing a quantity of at least one antibiotic substance in the infected zone.

With regard to the latter aspect, the spacer is able to cure the infection underway by releasing antibiotic in a focused manner and in infinitesimal quantities, while the application of even high doses of antibiotic, but with methods that do not provide for the use of spacers, e.g. washing the infected place with high-dosage antibiotics, does not allow obtaining the same results.

Studies conducted in the field have in fact shown that the bone tissue absorbs in a concentrated manner all the antibiotic molecules (even if only a few) daily released by the spacer. Naturally this is verified if the antibiotic is released by the spacer in contact with or adjacent to the bone tissue, in which case the quantity of antibiotic locally reaches the effective concentration for eradicating the infection. For this reason, it is essential that the spacer is extended over the entire area of the infection, thus intending that if the infected prosthesis is a long prosthesis, a long spacer will be used, and if the infected prosthesis is of short type, a short spacer will be used. If a short spacer is placed where a long prosthesis had previously been implanted, part of the bone would not be treated with antibiotic, thus allowing the bacteria to freely proliferate.

The spacer device according to the invention has a body shaped in a manner such to be couplable, in a substantially complementary manner, to the bone or joint seat to which it must be constrained.

For such purpose, by way of a non-limiting example, several possible configurations of a spacer device according to the present invention are illustrated in the enclosed figures.

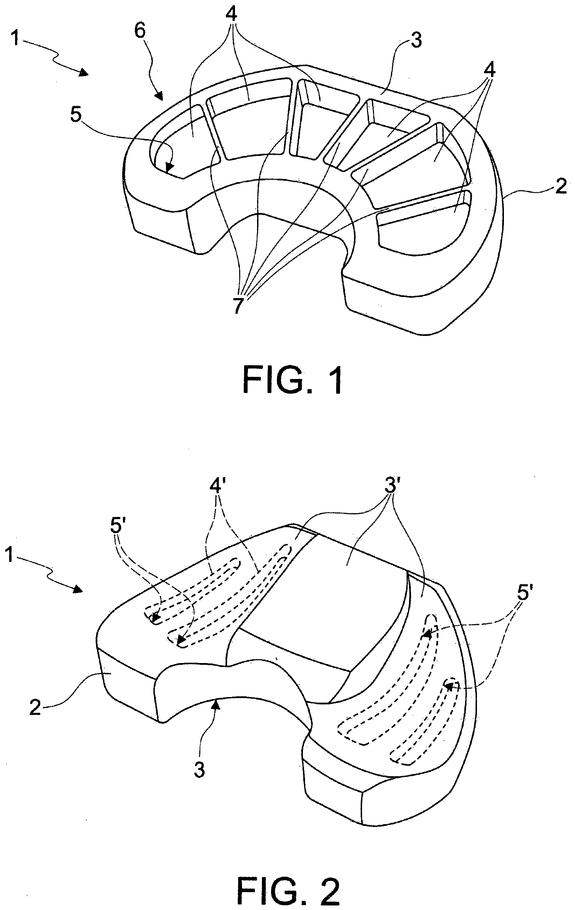

More in detail, by way of example, in figures 1 and 2 a version of the spacer device according to the present invention is illustrated that is shaped in order to be constrained to the tibial end of a knee joint.

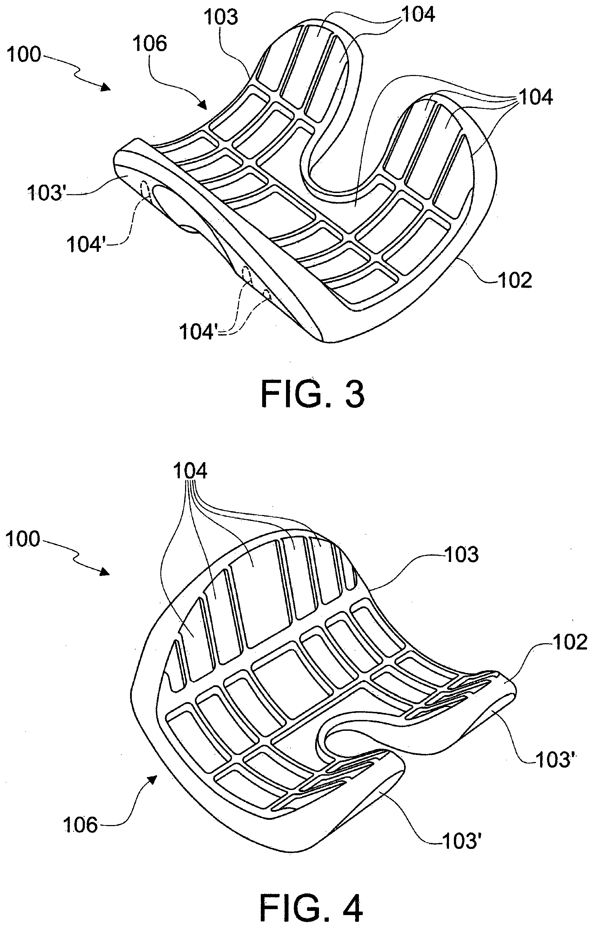

A further version of a spacer device according to the present invention is illustrated in figures 3 and 4, in which a spacer device is represented, provided in order to be associated with the femoral bone end of the knee joint.

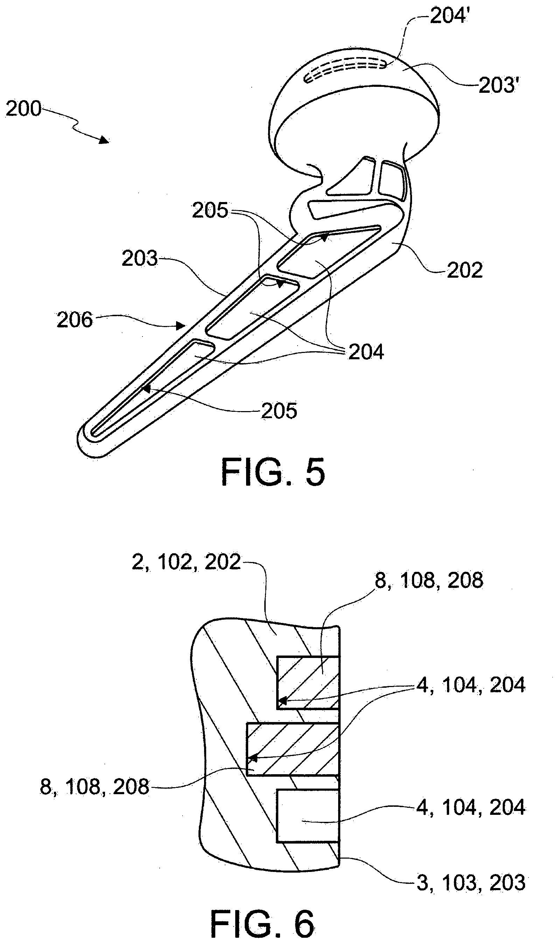

Another further version of a spacer device according to the present invention is illustrated in figure 5, in which a spacer device for the hip articulation is represented, provided in order to be associated with the upper femoral end at the articulation of the hip.

Nevertheless, further versions of the spacer device according to the present invention are possible, shaped differently from that illustrated in the enclosed figures, e.g. for use in the shoulder articulation or elbow articulation or for use in specific seats of the human body, without any limitation.

Hereinbelow, reference will be made to a spacer device for the tibial end of the knee joint, overall indicating it with the number 1.

The spacer device 1 comprises a body 2 configured in a substantially complementary manner to the bone or joint seat with which it must be associated or with part thereof.

The body 2 has at least one surface 3 for the coupling of the spacer device to the bone or joint seat to be treated or to a part thereof.

The at least one coupling surface 3 is therefore shaped substantially complementary to the bone or joint seat to which the spacer device is constrained, in order to facilitate the correct positioning and connection of the latter.

With reference to the embodiment illustrated in figures 1 and 2, the surface 3 is shaped for facilitating the coupling of the body 2 to the tibial end at the knee joint.

The body 2 has at least one recess 4.

The at least one recess 4 is adapted to contain a filling material containing at least one pharmaceutical or medical substance, as will be better described hereinbelow.

The spacer device of the invention has a plurality of recesses 4 positioned along the coupling surface 3.

Each recess 4 can be extended more or less deeply inside the body 2, as illustrated for example in figure 6.

In addition, the arrangement of the plurality of recesses along the coupling surface 3 can vary as a function of specific needs.

The extension of the recesses 4, can in any case vary as a function of the thickness of the body 2, as better described hereinbelow.

Each recess 4 determines a corresponding opening 5 through the coupling surface 3.

The at least one recess 4 affects or is positioned substantially along the entire surface 3 of the body 2, i.e. substantially affects the entire coupling surface of

the spacer device to the bone or joint seat to be treated or to part thereof.

As is better described hereinbelow, the at least one recess 4 forms a seat for housing a filling material comprising at least one pharmaceutical or medical substance in the body 2 of the spacer device 1.

In one version of the invention, the filling material is applied by the surgeon before the implant of the spacer device itself.

According to one aspect of the present invention, the substance to be introduced into the at least one recess 4, or only in some recesses 4 when they are more than one, comprises at least one pharmaceutical or medical substance for the treatment of the infection present in the bone or joint seat with which the spacer device 1 is associated.

The recesses 4, in practice, act as a store for the storage of at least one pharmaceutical or medical substance to be released inside the human body.

The total volume of the recesses 4 or of the recess 4 is thus suitable for the estimated period for the treatment of the infection underway in the seat with which the spacer device is associated.

Actually, a spacer device according to the present invention ensures the release of at least one pharmaceutical or medical substance for the entire period estimated for the treatment of the infection. The recesses 4 define an open cell structure 6 along the coupling surface 3.

More in detail, the open cell structure 6 comprises a plurality of cells adjacent to each other, corresponding to the recesses 4.

According to one version, the recesses 4 are adjacent to each other.

The open cells 6 or the recesses 4 are separated from each other by ribs 7 interposed between the recesses 4 themselves or between the cells 6 themselves.

The distance along with the mutual positioning between the single recesses 4 can vary as a function of the shape of the spacer device 1, and hence of the shape of its coupling surface 3.

The ribs 7, in addition to spacing and separating the recesses 4, act as reinforcement element for the body 2.

For such purpose, the recesses 4 can be made in the body 2 in a manner such that the ribs 7 are positioned at the portions of the spacer device 1 which, during use, are subjected to greater mechanical stresses, e.g. wear, bending, fatigue, etc.

According to the invention, the opening of the at least one recess 4 is wide with respect to its depth. In such a manner, the application of the filling material, comprising the at least one pharmaceutical or medical substance, by the surgeon inside the at least one recess 4 is facilitated, as will be better described hereinbelow.

According to the present invention, the body 2 is made of biologically compatible material, which is porous.

The biologically compatible material constituting the body 2 can be selected from among plastic materials, possible thermoformable, such as polymethylmethacrylate (PMMA), polyvinylchloride (PVC), polystyrene (PS), polyethylene (PE), ultrahigh molecular weight polyethylene (UHMWPE), high or low density polyethylene, etcetera, or non-polymer materials, ceramics, metals, metal alloys, organometallic compounds, and/or a combination of the same.

In one version of the present invention, the biologically compatible material is a bone cement with polymethylmethacrylate (PMMA) base.

In another version of the invention, the aforesaid biologically compatible material initially lacks pharmaceutical or medical substances.

In further second version, the aforesaid biologically compatible material comprises at least one pharmaceutical or medical substance.

According to a further version of the present invention, the biologically compatible material can be a ceramic cement, such as calcium sulfate known as plaster or CaSO₄, which in addition to solidifying in limited times is capable of releasing calcium ions.

In order to make the body 2, further materials of biocompatible type can nevertheless be used, with respect to that described above, without departing from the protective scope of the present invention.

According to one aspect of the present invention, the at least one recess 4, or overall the plurality of recesses 4, ensure the release of the at least one pharmaceutical or medical substance contained therein for a period at least equal to that estimated for the treatment of the infection.

For such purpose, the total volume of the recess/recesses 4 to be arranged in a spacer device according to the present invention is calculated as a function of different parameters, including the estimated quantity of at least one pharmaceutical or medical substance necessary for the aforesaid treatment period, the volume of the body 2, the extension of the coupling surface 3, the value of the stresses to which the spacer device is subjected during use, the possible presence or lack of presence of reinforcement cores inside the spacer device itself.

In principle, if a spacer device has a wide coupling surface 3 in relation to the volume of the body 2, then in the presence of a spacer device with a limited thickness, the at least one recess 4 can have limited depth with respect to the planar extension thereof.

As stated above, the shape of the at least one recess 4 can, in any case, vary and differ from that described above, as a function of specific use needs. For example, the single recesses 4 can have shapes that are different from each other, as a function of the shape of the body 2 and of the coupling surface 3. As stated above, the single recesses 4 can have different depths with respect to the coupling surface 3. For such purpose, in figure 6, a section view detail is illustrated of the body of a spacer device, according to the present invention, at the coupling surface 3.

In figure 6, three recesses 4 are schematically illustrated, by way of example, arranged adjacent to each other; such recesses extend through the body 2 with different depths, two of which are filled with at least one filling material.

According to one version of the present invention, the recesses 4 can be present along the coupling surface 3, distributed in a substantially uniform manner (see figures 1 and 3). In addition, the recesses 4 are made or affect substantially the entire coupling surface 3.

In such case, the body 2 has an open cell structure 6 with cells spaced from each other in a substantially uniform manner along the coupling surface 3.

According to a further version, the recesses 4 can be positioned in a non-uniform manner along the coupling surface 3.

If the spacer device is provided for use in an articulation of the human body, the same can comprise an articulation surface 3', capable of being articulated with a suitable bone seat or with a corresponding surface present in a spacer device of suitable configuration.

With reference to the spacer device 1 illustrated in figures 1 and 2, the articulation surface 3' is opposite the coupling surface 3.

The coupling surface 3 and the articulation surface 3' can be arranged in a different manner from each other, as a function of the shape of the spacer device itself.

The articulation surface 3' can, possibly, have at least one further recess 4'.

The at least one recess 4 or the further recesses 4', analogous to that described above regarding the recesses 4, identify an opening 5' or openings 5' through the articulation surface 3'.

The further recesses 4', illustrated with discontinuous dashed line in figure 2, are arranged along the articulation surface 3' aligned along the articulation direction.

Therefore, the further recesses 4' facilitate or at least do not obstruct the sliding between the articulation surface 3' and the surface of the joint bone seat or of the corresponding spacer device with which the spacer device 1 is articulated.

If the articulation, to which a spacer device 1 according to the present invention has to be applied, allows a bending and extension movement between two bone ends around a rotation axis, for example in the case of the knee joint or elbow joint, the further recesses 4' can be substantially orthogonal to such rotation axis, and oriented along the bending - extension direction (see figure 2).

The further recesses 4' thus oriented facilitate or do not obstruct the mobility of the articulation, since the articulation surface 3' has portions for the abutment with the bone end with which it is associated, thus ensuring the mobility of the articulation itself.

According to the present invention, the spacer device 1 comprises filling portions or inserts, overall indicated with the number 8 (see figure 6).

The filling portions or inserts 8 act as filling for the recesses 4, 4' present in the body 2.

According to the present invention, the filling portions or inserts 8 are made by using a filling material of hardening or solidifiable type.

The filling material according to the invention can be of hardening or solidifiable type.

According to the invention, the filling material is of the type that is not resorbable or degradable in the human body at least for the stay time of the device inside the human body itself. The filling material is therefore permanent.

Naturally, the pharmaceutical or medical substance, such as at least one antibiotic, inserted in the filling material is of soluble type, and therefore is released towards the bone tissue that is adjacent or in contact with the filling material in order to heal or at least prevent infection thereof.

In one version of the invention, the filling material can be prepared by the surgeon during the operating procedure.

Such filling material can lack pharmaceutical or medical substances and can be admixed with the same on the basis of the selection of the surgeon and of the patient needs.

In a further version of the invention, the aforesaid filling material can comprise at least one pharmaceutical or medical substance already arranged in the material that constitutes the filling material itself, and possibly can, in preparation step, be admixed with a further substance.

According to the present invention, the filling material, by virtue of the preparation and solidification step to which it is subjected, is porous.

The size of the pores present in the filling portions or inserts 8 is such to prevent, during use, bone regrowth from occurring inside the same and, therefore, inside the spacer device that comprises them, which as stated above is temporary.

One such configuration of the pores thus facilitates the subsequent removal of the spacer device itself from the treated bone or joint seat, once its curative function has been completed.

The pores have average dimensions smaller than 100 micron.

The spacer device 1 is configured in a manner such that when, during use, it is implanted in the human body, the filling portions or inserts 8 are in contact with the bone tissues to be treated.

According to one version, the filling portions or inserts 8 are made in a manner such to be flush with the coupling surface 3 and/or with the articulation surface 3'.

According to a further version, the filling portions or inserts 8 are made projecting from the coupling surface 3 and/or from the articulation surface 3'.

According to a further version, some of the filling portions or inserts 8 are formed flush with the coupling 3, or articulation 3' surfaces and others projecting from the coupling 3, or articulation 3' surfaces.

In figures 3 and 4, a further embodiment of a spacer device is illustrated, indicated overall with 100, of the type usable for the femoral end of the knee joint. Hereinbelow, the same components corresponding to those of the previously described embodiment will be indicated with the same reference numbers increased by 100.

The spacer device 100 overall differs from the preceding embodiment only for its shape, since it is provided to be associated with the lower femoral end.

The spacer device 100 is adapted to be articulated with the spacer device 1.

The spacer device 100 comprises a body 102 which has at least one coupling surface 103 associable with the femoral bone end at the knee joint. The body 102 comprises at least one recess 104 which identifies at least one respective opening 105 through the at least one coupling surface 103.

According to the present invention, the spacer device 100 comprises filling portions or inserts, overall indicated with the number 108 (see figure 6), for respective recesses 104.

The volume of the single recesses 104, and consequently of the respective filling portions 108, can vary, among other aspects, as a function of the position of the recesses 104 themselves along the at least one coupling surface 103 and of the shape of the body 102.

The spacer device 100 has an articulation surface 103' associable with a tibial abutment portion of the knee joint or with the articulation surface 3 of the spacer device 1. The articulation portion 103' is opposite the at least one coupling surface 103.

The articulation surface 103' may possibly have further recesses 104', illustrated in figure 3 with a discontinuous dashed line, with elongated form and oriented along the bending/extension direction of the knee joint.

In figure 5, a further embodiment of a spacer device according to the present invention is illustrated, usable in the hip articulation, indicated overall with 200.

Hereinbelow, the same components corresponding to those of the previously described embodiments will be indicated with the same reference numbers increased by 100.

The spacer device 200 differs from the preceding embodiments due to the shape of the body 202 which has a substantially elongated or stem portion, for the coupling with the upper femoral end, and a rounded or head end portion, associable with the acetabular cavity and adapted to be articulated therewith.

At the elongated portion, the body 202 has at least one coupling surface 203, along which the recesses 204 are made.

The recesses 204 identify respective openings 205 through the at least one coupling surface 203.

As described for the preceding embodiments, the at least one coupling surface 203 has an open cell structure 206, in which the cells correspond to the recesses 204.

According to the present invention, the spacer device 200 comprises filling portions or inserts, overall indicated with the number 208 (see figure 6), for at least some of the respective recesses 204.

The recesses 204 can have different depths from each other with respect to the coupling surface 203, as a function of specific needs.

The body 202 has an articulation surface 203' capable of being articulated with the acetabular seat of the hip articulation.

Analogous to that described for the preceding embodiments, the body 202 can comprise a further recess 204' or further recesses 204' arranged along the articulation surface 203'.

As stated above, the spacer device 1, 100, 200 according to the present invention comprises a body 2, 102, 202 provided with at least one recess 4, 104, 204 for housing a filling material and at least one pharmaceutical or medical substance for treating an infection of a bone or joint seat of the human body, in which said at least one recess 4, 104, 204 is arranged along at least one coupling surface 3, 103, 203 with the respective bone seat.

As stated above, such at least one insert 4, 104, 204 affects or is positioned substantially along the entire coupling surface 3, 103, 203 with the respective bone seat.

The body 2, 102, 202 can also comprise at least one further recess 4', 104', 204' at the articulation surface 3', 103', 203'.

In one version of the invention, such at least one insert 4', 104', 204' affects or is positioned substantially along the entire coupling surface 3', 103', 203' with the respective bone seat.

In one version of the invention, the spacer device 1, 100, 200 comprises at least one recess 4, 104, 204 in the coupling surface 3, 103, 203 but does not comprise any recess in the articulation surface 3', 103', 203'. The recesses 4, 4', 104, 104', 204, 204' allow a surgeon to arrange, in specific portions of the spacer device 1, 100, 200 which during use are directly in contact with specific portions of the bone tissues to be treated, filling portions 8, 108, 208 comprising such at least one substance, in a manner such that the same is released into the human body.

The surgeon can decide in which portions of the coupling surface 3, 103, 203 and/or of the articulation surface 3', 103', 203' of the spacer device 1, 100, 200 he/she will apply at least one pharmaceutical or medical substance.

In particular, the surgeon is facilitated in this step since the single recesses 4, 4', 104, 104', 204, 204' are independent from each other. Therefore, the at least one substance introduced into the same is confined, during the arrangement of the spacer device for its implant in the human body, inside the recesses themselves and, hence, in specific portions of the body 2, 102, 202.

As stated above, the total volume of the recesses 4, 4', 104, 104', 204, 204' can vary as a function of various parameters, including the shape of the spacer device itself and the extension of the coupling surface 3, 103, 203.

Generally, the total volume of the recesses 4, 4', 104, 104', 204, 204' can be comprised between 1%-80%, with respect to the total volume of the body 2, 102, 202.

The higher percentage is obtainable when the material that composes the body of the spacer device is *per se* resistant to loads and to stresses, even if provided with many recesses (and thus when the percentage of volume occupied by the material of the body is lower than the "empty" volume relative to the recesses).

This can occur, for example, in one version of the invention, when the material which constitutes the body of the spacer device is a metal or a material with similar mechanical strength performances.

Alternatively, the total volume of the recesses 4, 4', 104, 104', 204, 204' can be comprised between 2% and 30% of the total volume of the body 2, 102, 202. Such percentage - lower than that described above - can for example be used in one version of the invention, when the body is made of a plastic material, e.g. PMMA.

One characteristic of the spacer device according to the present invention is that, even in the presence of a high volume of recesses, the same do not alter the final shape of the device itself.

Therefore, the spacer device maintains a predefined and preformed overall shape - even in the presence of the at least one recess - in a manner such that the final form thereof has the advantages of the preformed devices of conventional type.

As stated above, in fact, the advantages conferred by the present invention are associated with the need to arrange a disposable and/or temporary spacer device with predefined and correct shape and size, without the risk that the surgeon - having to make the filling of the recesses present in the spacer device directly *in situ* - will obtain a shape that is irregular or incompatible with the actual anatomic or articulation needs of the patient.

Indeed, the recesses, when they are filled with the filling material, recreate the overall or final form of the spacer device without requiring complicated or further working by the surgeon, who otherwise would be obliged to verify the suitability of the surfaces recreated thereby with the filling material, with respect to the actual anatomic and/or articulation needs of the patient.

For example, in one version of the invention, the recesses can be filled with the filling material or closed with the same in order to define a flat outer surface.

For such purpose, the opening of such recesses or the outer surface thereof, defines a surface which substantially lies on the same plane.

In such case, the surface in question is flat.

In such case, the opening of the at least one recess or the outer surface of the at least one filling portion or insert is flush with the surface of the body of the spacer device or is flush with the outer edge of the ribs 7, 107, 207.

Consequently, the outer surface of the recess or of the filling portions or inserts 8, 108, 208, which are formed following the filling of the recesses with the filling material, is flat or not curved.

In such mode, when the surgeon fills such recesses, he/she can also level the outermost surface of the filling material contained therein with a spatula, in any case obtaining a defined final overall shape of the spacer device, reflecting the anatomic and/or articulation needs of the patient.

For example, when the at least one recess is positioned in a curved surface of the spacer device, the flat outer surface of the recess or of the at least one filling portion or insert 8, 108, 208 do not interfere with the curved progression of the surface on which they are found to be positioned. Therefore, the outer surface will have size adapted for such purpose.

The area occupied by the outer surface of the at least one recess or of each recess, in one version of the invention, has values comprised between 10% and 80%, when positioned on a substantially flat surface and/or on an at least partially curved surface of the spacer device in question.

In one version, the area affected by the at least one recess 4, 104, 204, 4', 104', 204' corresponds to about 80% of the area of the respective coupling surface 3, 103, 203, 3', 103', 203' or comprised between 70% and 90% or between 60% and 80% of the same or according to percentages that lie between the above values.

The present invention then regards a method for making a spacer device 1, 100, 200.

The aforesaid method initially provides for providing a body 2, 102, 202 shaped in a substantially complementary manner to the bone or joint seat with which it must be associated or with part thereof.

Subsequently, the method provides for filling the at least one recess 4, 104, 204 or at least some of the recesses 4, 104, 204 by introducing at least one filling material inside the same.

If present, also the at least one recess 4', 104', 204' or at least some of the further recesses 4', 104', 204' can be filled with a filling material.

Such filling material comprises at least one pharmaceutical or medical substance.

As stated above, typically, the usable filling material is of solidifiable type, such as bone cement, e.g. of acrylic type, such as polymethylmethacrylate.

In one version of the invention, the filling material comprises a solidifiable ceramic bone cement.

Following the hardening of the filling material in the body 2, 102, 202, filling portions or inserts 8, 108, 208 are formed which at the end of the solidification actually form a single body with the body 2, 102, 202 itself.

The spacer device 1, 100, 200 thus formed therefore has a body 2, 102, 202 composed of a first material, and filling portions or inserts 8, 108, 208 composed of at least one second material.

According to one version, the filling material with which the filling portions 8, 108, 208 are composed can differ from the material that constitutes the body 2, 102, 202.

According to the invention, the filling material comprises at least one first pharmaceutical or medical substance.

According to a further version, the filling material can correspond to that of the body 2, 102, 202.

If, however, a material is selected for the body 2, 102, 202 that is different from that of the filling material, the same could for example have the following combinations.

The body can be obtained from molding of plastic materials by means of a thermoplastic press.

The plastic materials could be selected from among, as stated above, PE, UHMWPE, PP, PA, PMMA, PEEK, etcetera.

The selected plastic material is suitable for being implanted in the human body for a long time.

Alternatively, the body could be made of molded ceramic and glaze-fired as occurs for medical ceramics such as alumina, zirconia, etc.

In addition, the body could be made of metal or metal alloys with particular anti-bacterial activity (silver, etc.), molded for example with the MIM technique.

In these cases, the filling material is always bone cement or acrylic bone cement or PMMA.

One of the advantages of such examples is that the material of the containment body is not steel. Steel, as is known, forms a site where the bacteria can be housed and survive. Therefore, a spacer - which is positioned in an infected articulation - must not even minimally have exposed parts made of steel. If this was the case, bacteria would quickly settle on the exposed steel; such bacteria, possibly protected by their "glycocalyx", might resist the antibiotic released by the nearby spacer device (e.g. by its coat or body of bone cement).

If however the steel is perfectly immersed in the antibiotic cement, this risk does not exist.

The material forming the body 2, 102, 202 must be insensitive to bacteria colonization and this can be done, according to one embodiment, by inserting antibiotics or antimicrobials in the thermo-molded plastic material or in the ceramic or by using antibacterial metals such as silver.

The disposable temporary spacer device 1, 100, 200 according to the present invention is ready for its use following the hardening of the filling material introduced inside the recesses 4, 104, 204 or 4', 104', 204'.

The at least one pharmaceutical or medical substance present in the filling material can be gradually released, and in a substantially uniform manner, through the at least one coupling surface 3, 103, 203 and/or through the at least one articulation surface 3', 103', 203' of the spacer device 1, 100, 200.

As stated above, the recesses act as a tank for the storage of a suitable reserve of at least one pharmaceutical or medical substance in the body 2, 102, 202 as a function of the period estimated for the use of the spacer device 1, 100, 200.

In practice, the spacer device 1, 100, 200 allows the release, through the coupling surface 3, 103, 203 and/or through the at least one articulation surface 3', 103', 203', of at least one pharmaceutical or medical substance during the entire period estimated for the treatment of the infection or, possibly, for a longer time period.

In addition, the various recesses are independent from each other, and allow storing one or more pharmaceutical or medical substances, different from each other, maintaining them separate in specific portions of the body 2, 102, 202.

The given definition of "porous" element, present in the present discussion, can be substituted by "semipermeable", without departing from the protective scope of the present invention.

According to a further aspect of the present invention, the spacer device 1, 100, 200 can comprise at least one specific diagnostic or measurement device, not illustrated in the figures, housed inside one of the recesses 4, 4', 104, 104', 204, 204'.

By way of example, the spacer device 1, 100, 200 could comprise a biomedical/biological micro-electromechanical system, such as a bio-sensor, capable of carrying out detections of chemical-physical type. One such bio-sensor, which corresponds to a chip, could comprise a miniaturized circuit in turn comprising an accelerometer and/or a thermometer and/or a load cell and/or sensors adapted to detect further physical entities of different type.

The chip to be associated inside a spacer device 1, 100, 200 can be selected as a function of specific use needs and of the type of detections to be carried out.

By associating such chip with the spacer device 1, 100, 200 it is therefore possible to detect the use conditions of the spacer device 1, 100, 200 itself, with reference for example to the accelerations and/or to the static or dynamic loads to which it is subjected, to the temperature of the bone seat or joint seat in which it is implanted, etcetera.

According to a further aspect, the bio-sensor could comprise an integrated interface for transferring the detected data.

By way of example, the bio-sensor could comprise data transmission means for allowing the real-time detection of the use conditions of the spacer device 1, 100, 200 with which it is associated, thus allowing the monitoring of the operation of the spacer device 1, 100, 200 itself.

As can be understood, at least in one version of the invention, the filling material is viscous, so as to be able to fill the at least one recess 4, 104, 204, 4', 104', 204' without accidentally exiting therefrom. Indeed, the surgeon as stated above applies such material to the recess or recesses of interest, and the filling material must remain in position, being solidified in the recess where it was applied.

In one version of the invention, the recesses have dimensions (width and length) not smaller than 5 mm x 5 mm or, in case of circular recesses, a diameter with dimensions not smaller than 5 mm.

Indeed, the recesses according to the present invention must have dimensions such to be able to be filled with filling material, which as stated is a viscous fluid.

In one version of the invention, the filling material is not a liquid.

According to the invention, the recesses, or the openings delimited by the recesses, have a shape in plan view that is substantially polygonal, i.e. square, rectangular, triangular or trapezoidal, possibly with rounded edges.

In such case, the recesses have a shape substantially corresponding to the area of the coupling surface where they are positioned.

In addition, in one version of the invention, the ribs which delimit the recesses are substantially rectilinear and have a thickness smaller than the width of the adjacent recesses.

The invention thus conceived is susceptible of numerous modifications and variants, all falling within the scope of the inventive concept.

The characteristics presented for one version or embodiment can be combined with the characteristics of another version or embodiment, without departing from the protective scope of the present invention.

## Claims

1. Spacer device, of temporary and/or disposable type, implantable in the human body for the treatment of a bone seat or a joint seat affected by infection, comprising a body (2, 102, 202) configured in a substantially complementary manner to said bone seat or joint seat with which it must be associated in use, said body (2, 102, 202) being provided with at least one coupling surface (3, 103, 203) of said spacer device with a bone seat or joint seat of the human body, wherein said body (2, 102, 202) comprises at least one recess (4, 104, 204, 4', 104', 204') for housing a filling material comprising at least one pharmaceutical or medical substance, positioned along said coupling surface (3, 103, 203) and defining at least one opening (5, 105, 205, 5', 105', 205') through said coupling surface (3, 103, 203), said body (2, 102, 202) comprises a plurality of said at least one recess (4, 104, 204) defining an open cell structure (6, 106, 206) along said coupling surface (3, 103, 203), wherein said open cell structure (6, 106, 206) comprises a plurality of cells adjacent to one another, corresponding to said recesses (4, 104, 204), said recesses (4, 104, 204, 4', 104', 204') have ribs (7) that define the perimeter of said at least one recess (4, 104, 204, 4', 104', 204'), wherein said recesses (4, 104, 204, 4', 104', 204') affect substantially the entire coupling surface (3, 103, 203), wherein said filling material is of the setting or solidifiable type, wherein said body (2, 102, 202) of said spacer device (1, 100, 200) is made of a porous biologically compatible material, wherein said body (2, 102, 202) comprises at least one filling portion or insert (8, 108, 208), comprising said filling material, for filling at least one respective recess (4, 104, 204, 4', 104', 204'), wherein said filling material is of permanent type and wherein said at least one filling portion or insert (8, 108, 208) is flush with or projects with respect to said coupling surface (3, 103, 203), **characterised in that** each of said recesses (4, 104, 204, 4', 104', 204') has a shape in plan view that is substantially rectangular, triangular or trapezoidal, wherein said at least one opening (5, 105, 205, 5', 105', 205') of said at least one recess (4, 104, 204, 4', 104', 204') is wide with respect to the depth of said at least one recess (4, 104, 204, 4', 104', 204'), said at least one filling portion or insert (8, 108, 208) has pores, with pores having average dimensions smaller than 100 micron, at least one of said filling portions or inserts (8, 108, 208) comprises a filling material comprising at least one first pharmaceutical or medical substance and at least one other of said filling portions or inserts (8, 108, 208) comprises a filling material comprising a second pharmaceutical or medical substance different from said first pharmaceutical or medical substance, such that the various recesses allow separate storing of said first and second pharmaceutical or medical substances.

2. Spacer device according to claim 1, wherein the total volume of said at least one recess (4, 104, 204) provided in said body (2, 102, 202) is comprised between 1 and 80% or between 2% and 30% or between intermediate percentages between the aforementioned values with respect to the total volume of said body (2, 102, 202) or of said spacer device (1, 100, 200) and wherein the area of said at least one recess (4, 104, 204) corresponds to about 80% of the area of the respective coupling surface (3, 103, 203) or it is comprised between 70% and 90% or between 60% and 80% of the area of the coupling surface (3, 103, 203) or among percentages intermediate between the above-mentioned values with respect to the area of the coupling surface (3, 103, 203) .

3. Spacer device according to claim 1 or 2, wherein said at least one filling portion or insert (8, 108, 208) has a flat or planar outer surface.

4. Spacer device according to any one of the previous claims, wherein said body (2, 102, 202) has an articulation surface (3', 103', 203'), capable of articulating with a suitable bone seat or joint seat or with a corresponding surface present in a spacer device of suitable configuration.

5. Spacer device according to the previous claim, wherein said articulation surface (3', 103', 203') has at least one recess (4', 104', 204') defining a respective opening (5', 105', 205') along said articulation surface (3', 103', 203').

6. Method for making a spacer device according to any one of the preceding claims, comprising the steps of:
providing said body (2, 102, 202) of said spacer device (1, 100, 200),
filling said at least one recess (4, 104, 204) with said filling material comprising at least one first pharmaceutical or medical substance and filling at least one further of said recesses (4, 104, 204) with the other said filling material comprising said second pharmaceutical or medical substance different from said first pharmaceutical or medical substance,
waiting for a time in which said filling material sets,
wherein said step of filling said recesses (4, 104, 204) comprises:
introducing said filling materials in said recesses (4, 104, 204) to form filling portions or inserts (8, 108, 208),
levelling or making flush said filling materials, or possibly their outer surface, with respect to said coupling surface (3, 103, 203) of said body (2, 102, 202), wherein said filling portions or inserts (8, 108, 208) are flush with or project with respect to said coupling surface (3, 103, 203).

## Patentansprüche

1. Abstandsvorrichtung vom temporären und/oder entsorgbaren Typ, die in den menschlichen Körper zur Behandlung eines Knochensitzes oder eines Gelenksitzes, der von einer Infektion betroffen ist, implantierbar ist, umfassend einen Körper (2, 102, 202), der in einer im Wesentlichen komplementären Weise zu dem besagten Knochensitz oder Gelenksitz konfiguriert ist, mit dem er bei der Verwendung verbunden werden muss, wobei der besagte Körper (2, 102, 202) mit mindestens einer Kopplungsfläche (3, 103, 203) der besagten Abstandsvorrichtung mit einem Knochensitz oder Gelenksitz des menschlichen Körpers versehen ist, worin der besagte Körper (2, 102, 202) mindestens eine Aussparung (4, 104, 204, 4', 104', 204') zur Aufnahme eines Füllmaterials umfasst, das mindestens eine pharmazeutische oder medizinische Substanz umfasst, die entlang der besagten Kopplungsfläche (3, 103, 203) angeordnet ist und mindestens eine Öffnung (5, 105, 205, 5', 105', 205') durch die besagte Kopplungsfläche (3, 103, 203) definiert, wobei der besagte Körper (2, 102, 202) eine Vielzahl der besagten mindestens einen Aussparung (4, 104, 204) umfasst, die eine offene Zellstruktur (6, 106, 206) entlang der besagten Kopplungsfläche (3, 103, 203) definiert, worin die besagte offene Zellstruktur (6, 106, 206) eine Vielzahl von aneinander angrenzenden Zellen umfasst, die den besagten Aussparungen (4, 104, 204) entspricht, wobei die besagten Aussparungen (4, 104, 204, 4', 104', 204') Rippen (7) aufweisen, die den Umfang der besagten mindestens einen Aussparung (4, 104, 204, 4', 104', 204') definieren, worin die besagten Aussparungen (4, 104, 204, 4', 104', 204') im Wesentlichen die gesamte Kopplungsfläche (3, 103, 203) betreffen, worin das besagte Füllmaterial vom erhärtenden oder verfestigbaren Typ ist, worin der besagte Körper (2, 102, 202) der besagten Abstandsvorrichtung (1, 100, 200) aus einem porösen, biologisch kompatiblen Material besteht, worin der besagte Körper (2, 102, 202) mindestens einen Füllabschnitt oder Einsatz (8, 108, 208) umfasst, der das besagte Füllmaterial umfasst, um mindestens eine jeweilige Aussparung (4, 104, 204, 4', 104', 204') zu füllen, worin das besagte Füllmaterial vom permanenten Typ ist und worin der besagte mindestens eine Füllabschnitt oder Einsatz (8, 108, 208) mit der besagten Kopplungsfläche (3, 103, 203) bündig ist oder in Bezug auf diese vorsteht, **dadurch gekennzeichnet, dass** jede der besagten Aussparungen (4, 104, 204, 4', 104', 204') in der Draufsicht eine Form hat, die im Wesentlichen rechteckig, dreieckig oder trapezförmig ist, worin die besagte mindestens eine Öffnung (5, 105, 205, 5', 105', 205') der besagten mindestens einen Aussparung (4, 104, 204, 4', 104', 204') in Bezug auf die Tiefe der besagten mindestens einen Aussparung (4, 104, 204, 4', 104', 204') breit ist, wobei der besagte mindestens eine Füllabschnitt oder Einsatz (8, 108, 208) Poren aufweist, mit Poren, deren durchschnittliche Größen kleiner als 100 Mikron sind, wobei mindestens einer der besagten Füllabschnitte oder Einsätze (8, 108, 208) ein Füllmaterial umfasst, das mindestens eine erste pharmazeutische oder medizinische Substanz umfasst, und mindestens ein anderer der besagten Füllabschnitte oder Einsätze (8, 108, 208) ein Füllmaterial umfasst, das eine zweite pharmazeutische oder medizinische Substanz umfasst, die sich von der besagten ersten pharmazeutischen oder medizinischen Substanz unterscheidet, so dass die verschiedenen Aussparungen eine getrennte Lagerung der besagten ersten und zweiten pharmazeutischen oder medizinischen Substanzen ermöglichen.

2. Abstandsvorrichtung nach Anspruch 1, worin das Gesamtvolumen der besagten mindestens einen Aussparung (4, 104, 204), die in dem besagten Körper (2, 102, 202) vorgesehen ist, zwischen 1 und 80 % oder zwischen 2 % und 30 % oder zwischen zwischenliegenden Prozentsätzen, die zwischen den vorgenannten Werte in Bezug auf das Gesamtvolumen des besagten Körpers (2, 102, 202) oder der besagten Abstandsvorrichtung (1, 100, 200) liegen, liegt und worin der Bereich der besagten mindestens einen Aussparung (4, 104, 204) etwa 80 % des Bereichs der jeweiligen Kopplungsfläche (3, 103, 203) entspricht oder zwischen 70 % und 90 % oder zwischen 60 % und 80 % des Bereichs der Kopplungsfläche (3, 103, 203) oder unter Prozentsätzen, die zwischen den oben genannten Werten in Bezug auf den Bereich der Kopplungsfläche (3, 103, 203) zwischenliegend sind, liegen.

3. Abstandsvorrichtung nach Anspruch 1 oder 2, worin der besagte mindestens eine Füllabschnitt oder Einsatz (8, 108, 208) eine flache oder ebene Außenfläche aufweist.

4. Abstandsvorrichtung nach irgendeinem der vorangegangenen Ansprüche, worin der besagte Körper (2, 102, 202) eine Gelenkfläche (3', 103', 203') aufweist, die fähig ist, mit einem geeigneten Knochensitz oder Gelenksitz oder mit einer entsprechenden Fläche, die in einem Abstandshalter geeigneter Konfiguration vorhanden ist, gelenkig verbunden zu werden.

5. Abstandsvorrichtung nach dem vorangehenden Anspruch, worin die besagte Gelenkfläche (3', 103', 203') mindestens eine Aussparung (4', 104', 204') aufweist, die eine jeweilige Öffnung (5', 105', 205') entlang der besagten Gelenkfläche (3', 103', 203') definiert.

6. Verfahren zur Herstellung einer Abstandsvorrichtung nach irgendeinem der vorangegangenen Ansprüche, umfassend die folgenden Schritte des:
Bereitstellens des besagten Körpers (2, 102, 202) der besagten Abstandsvorrichtung (1, 100, 200),
Füllens der besagten mindestens einen Aussparung (4, 104, 204) mit dem besagten Füllmaterial, das mindestens eine erste pharmazeutische oder medizinische Substanz umfasst, und Füllens mindestens einer weiteren der besagten Aussparungen (4, 104, 204) mit dem anderen besagten Füllmaterial, das die besagte zweite pharmazeutische oder medizinische Substanz, die sich von der besagten ersten pharmazeutischen oder medizinischen Substanz unterscheidet, umfasst,
Abwartens einer Zeit, in der das besagte Füllmaterial erhärtet,
worin der besagte Schritt des Füllens der besagten Aussparungen (4, 104, 204) umfasst:
Einbringen der besagten Füllmaterialien in die besagten Aussparungen (4, 104, 204), um Füllabschnitte oder Einsätze (8, 108, 208) zu bilden,
Nivellieren oder Bündigmachen der besagten Füllmaterialien, oder möglicherweise ihrer Außenfläche, in Bezug auf die besagte Kopplungsfläche (3, 103, 203) des besagten Körpers (2, 102, 202), worin die besagten Füllabschnitte oder Einsätze (8, 108, 208) mit der besagten Kopplungsfläche (3, 103, 203) bündig sind oder in Bezug auf diese vorstehen.

## Revendications

1. Dispositif d'espacement, de type temporaire et/ou jetable, implantable dans le corps humain pour le traitement d'un siège osseux ou d'un siège articulaire affecté par une infection, comprenant un corps (2, 102, 202) configuré d'une manière sensiblement complémentaire au siège osseux ou siège articulaire auquel il doit être associé en usage, ledit corps (2, 102, 202) étant pourvu d'au moins une surface d'accouplement (3, 103, 203) dudit dispositif d'espacement avec un siège osseux ou un siège articulaire du corps humain, ledit corps (2, 102, 202) comprenant au moins un évidement (4, 104, 204, 4', 104', 204') pour loger un matériau de remplissage comprenant au moins une substance pharmaceutique ou médicale, positionné le long de ladite surface d'accouplement (3, 103, 203) et définissant au moins une ouverture (5, 105, 205, 5', 105', 205') à travers ladite surface d'accouplement (3, 103, 203), ledit corps (2, 102, 202) comprend une pluralité dudit au moins un évidement (4, 104, 204) définissant une structure à cellules ouvertes (6, 106, 206) le long de ladite surface d'accouplement (3, 103, 203), dans laquelle ladite structure à cellules ouvertes (6, 106, 206) comprend une pluralité de cellules adjacentes les unes aux autres, correspondant auxdits évidements (4, 104, 204), lesdits évidements (4, 104, 204, 4', 104', 204') ont des nervures (7) qui définissent le périmètre dudit au moins un évidement (4, 104, 204, 4', 104', 204'), dans lequel lesdits évidements (4, 104, 204, 4', 104', 204') affectent sensiblement toute la surface d'accouplement (3, 103, 203), ledit matériau de remplissage étant du type durcissable ou solidifiable, ledit corps (2, 102, 202) dudit dispositif d'espacement (1, 100, 200) étant constitué d'un matériau poreux biologiquement compatible, ledit corps (2, 102, 202) comprenant au moins une partie de remplissage ou insert (8, 108, 208), comprenant ledit matériau de remplissage, pour le remplissage d'au moins un évidement respectif (4, 104, 204, 4', 104', 204'), dans lequel ledit matériau de remplissage est de type permanent et dans lequel ladite au moins une partie de remplissage ou insert (8, 108, 208) est alignè avec ou fait saillie par rapport à ladite surface d'accouplement (3, 103, 203), **caractérisé en ce que** chacun desdits évidements (4, 104, 204, 4', 104', 204') a une forme en plan qui est sensiblement rectangulaire, triangulaire ou trapézoïdale, dans laquelle ladite au moins une ouverture (5, 105, 205, 5', 105', 205') dudit au moins un évidement (4, 104, 204, 4', 104', 204') est large par rapport à la profondeur dudit au moins un évidement (4, 104, 204, 4', 104', 204'), ladite au moins une partie de remplissage ou insert (8, 108, 208) a des pores, les pores ayant des dimensions moyennes inférieures à 100 microns, au moins l'une desdites parties de remplissage ou inserts (8, 108, 208) comprend un matériau de remplissage comprenant au moins une première substance pharmaceutique ou médicale et au moins une autre desdites parties de remplissage ou inserts (8, 108, 208) comprend un matériau de remplissage comprenant une deuxième substance pharmaceutique ou médicale différente de ladite première substance pharmaceutique ou médicale, de sorte que les différents évidements permettent un stockage séparé desdites première et deuxième substances pharmaceutiques ou médicales.

2. Dispositif d'espacement selon la revendication 1, dans lequel le volume total dudit au moins un évidement (4, 104, 204) prévu dans ledit corps (2, 102, 202) est compris entre 1 et 80 % ou entre 2 et 30 % ou entre des pourcentages intermédiaires entre les valeurs susmentionnées par rapport au volume total dudit corps (2, 102, 202) ou dudit dispositif d'espacement (1, 100, 200) et dans lequel l'aire dudit au moins un évidement (4, 104, 204) correspond à environ 80 % de l'aire de la surface d'accouplement respective (3, 103, 203) ou elle est comprise entre 70 % et 90 % ou entre 60 % et 80 % de l'aire de la surface d'accouplement (3, 103, 203) ou parmi les pourcentages intermédiaires entre les valeurs mentionnées ci-dessus par rapport à l'aire de la surface d'accouplement (3, 103, 203).

3. Dispositif d'espacement selon la revendication 1 ou 2, dans lequel ladite au moins une partie de remplissage ou insert (8, 108, 208) a une surface extérieure plate ou plane.

4. Dispositif d'espacement selon l'une quelconque des revendications précédentes, dans lequel ledit corps (2, 102, 202) a une surface d'articulation (3', 103', 203'), capable de s'articuler avec un siège osseux ou un siège articulaire approprié ou avec une surface correspondante présente dans un dispositif d'espacement de configuration appropriée.

5. Dispositif d'espacement selon la revendication précédente, dans lequel ladite surface d'articulation (3', 103', 203') comporte au moins un évidement (4', 104', 204') définissant une ouverture respective (5', 105', 205') le long de ladite surface d'articulation (3', 103', 203').

6. Procédé de fabrication d'un dispositif d'espacement selon l'une quelconque des revendications précédentes, comprenant les étapes consistant à :
fournir ledit corps (2, 102, 202) dudit dispositif d'espacement (1, 100, 200),
remplir ledit au moins un évidement (4, 104, 204) avec ledit matériau de remplissage comprenant au moins une première substance pharmaceutique ou médicale et remplir au moins un autre desdits évidements (4, 104, 204) avec ledit autre matériau de remplissage comprenant ladite deuxième substance pharmaceutique ou médicale différente de ladite première substance pharmaceutique ou médicale, attendre un temps pendant lequel ledit matériau de remplissage durcit,
dans lequel ladite étape de remplissage desdits évidements (4, 104, 204) comprend :
introduire lesdits matériaux de remplissage dans lesdits évidements (4, 104, 204) pour former des parties de remplissage ou inserts (8, 108, 208),
niveller ou aligner lesdits matériaux de remplissage, ou éventuellement de leur surface extérieure, par rapport à ladite surface d'accouplement (3, 103, 203) dudit corps (2, 102, 202), dans lequel lesdites parties de remplissage ou inserts (8, 108, 208) are alignès ou font saillie par rapport à ladite surface d'accouplement (3, 103, 203).
